# EUROPEAN PATENT SPECIFICATION

(11) **EP 2 704 588 B1**
(45) Date of publication and mention of the grant of the patent: **20.01.2016**
(21) Application number: 11720068.3
(22) Date of filing: 03.05.2011
(51) Int. Cl.: A23J 3/34, A23L 1/305, A23L 1/23

(54) **A HYDROLYSATE OF A PROTEIN SUBSTRATE AND A PROCESS FOR PRODUCING THEREOF**
HYDROLYSAT EINES PROTEINSUBSTRATS UND VERFAHREN ZU SEINER HERSTELLUNG
HYDROLYSAT D'UN SUBSTRAT PROTÉIQUE ET SON PROCÉDÉ DE PRODUCTION

(43) Date of publication of application: 12.03.2014
(73) Proprietor: Nestec S.A., 1800 Vevey (CH)
(72) Inventor: LIM, Bee Gim, Singapore 640719 (SG); HO DAC, Thang, CH-1052 Le-mont-sur-lausanne (CH)
(74) Representative: Mollet, Beat Max
(86) International application number: PCT/EP2011/057040
(87) International publication number: WO 2012/149959

(56) References cited:
- EP-A1- 0 824 873
- EP-A1- 1 967 524
- WO-A1-94/25580
- WO-A1-97/00078
- WO-A1-2009/076996
- FR-A1- 2 927 336
- BACA D. REBECA, M. T. PENA-VERA AND M. DIAZ-CASTANEDA: "Production of Fish Protein Hydrolysates with Bacterial Proteases; Yield and Nutritional Value", JOURNAL OF FOOD SCIENCE, [Online] vol. 56, no. 2, 1991, pages 309-314, XP002670224, Retrieved from the Internet: URL:http://onlinelibrary.wiley.com/doi/10. 1111/j.1365-2621.1991.tb05268.x/pdf> [retrieved on 2012-02-22]
- GEORGIOS LALASIDIS, STIG BOSTROM, LARS BORJE SJOBERG: "Low molecular weight enzymatic fish protein hydrolysates: chemical composition and nutritive value", J. AGRIC. FOOD CHEM., [Online] vol. 26, no. 3, 1978, pages 751-756, XP002670225, DOI: 10.1021/jf60217a045 Retrieved from the Internet: URL:http://pubs.acs.org/doi/abs/10.1021/jf 60217a045> [retrieved on 2012-02-22]

## Description

The present invention provides a novel hydrolysate of a substrate comprising animal protein and a process for producing the hydrolysate.

Fish sauces or fermented fish products are widely consumed as seasonings or cooking aids in Asian countries such as Japan, China and Southeastern countries such as Vietnam, Cambodia or Thailand. They are also widely consumed in Europe and North America. Fish sauces and fermented fish products are traditionally prepared through fermentation of fish in the presence of salt. Thereby, the presence of salt acts as an important hurdle against food spoilage and toxic microorganisms in those processes and hence for assuring the microbial safety of the fermented or hydrolysed food products.

One of the traditional processes is based on autolysis, where endogenous enzymes are released from the substrate, e.g. the fish meat, and then contribute to the hydrolysis of the substrate itself. This naturally occurring process usually takes a long period of time which can be from 6 to 12 months in order to fully obtain an acceptable yield and sensory property in terms of flavour, aroma and texture of the resulting hydrolysate. In view of the relatively long processing time, alternative solutions have been proposed and commercially implemented to reduce the production time for such hydrolysed food products.

One of the alternative solutions is acid hydrolysis, whereby for example hydrochloric acid is used to hydrolyse the substrate proteins. The process is substantially shorter and only takes a few hours to completion. Nonetheless, this process is considered harsh as it may destroy some of the individual amino acids and/or vitamins. Furthermore, hydrolysates produced by this method usually have less aroma and taste than traditionally produced products. Additionally, depending on the types of substrate and acid used, some chloro-compounds may arise during the process, which is not always desired.

Another commonly known hydrolysis process is the enzymatic hydrolysis, where enzyme-rich components are added to the starting material. Proteolytic enzymes from animal organs such as pancreatin and pepsin, and from plants such as bromelain of pineapple stems and papain of unripe papaya, have been used for this purpose. The process usually takes a few weeks and a fish sauce can for example be recovered after 3 to 4 weeks. The enzymatic hydrolysis process is more gentle than the acid hydrolysis. However, the sensory characteristics of the final hydrolysate are still inferior to the traditional methods, as the end product generally possesses a strong bitter taste.

In the traditional methods, high amounts of salts are added to the process used to produce fish sauces or fermented fish products. The high salt concentration guarantees on one hand the microbial food safety of the process, as food spoilage microorganisms are strongly inhibited in their growth. But it inhibits on the other hand also strongly the activity of the proteolytic enzymes. As such, the process for hydrolysing proteins from for example a fish source is hence far from optimal. Furthermore, the salt content of the final product is consequently high as well, which is not always desired.

JP4197153 discloses a process to producing a hydrolysate of an animal proteinaceous material with a mold and/or a Koji yeast with reduced salt content by adding an alcohol to the hydrolysis process. This allows a.o. to reduce bacterial and other spoilage contaminations during the hydrolysis process despite the absence of salt. However, the presence of alcohol may not be desired in such a final food product.

WO2009/076996 discloses a process of preparing a hydrolysate of an edible animal or fish meat in the presence of an Apergillus fermented Koji in an environment with less than 2% salt content. The hydrolysis process typically takes 1 to several days before a product with a satisfactory good flavour note for e.g. a fermented fish sauce develops. However, it would be desirable to have a process to still produce more intense flavours from the hydrolysed animal meat substrates, while at the same time reducing the time needed for the hydrolysis.

WO 94/25580 A1 discloses a method for hydrolysing a protein by incubating with a proteolytic enzyme preparation.

In light of the above, there is a persisting need in the industry to introduce new hydrolysates with more intense and different flavour and aroma profiles, while maintaining the low level of salt in the process. Furthermore, it would be desirable to reduce the production time for those new hydrolysates.

Therefore, the object of the present invention is to improve the state of the art and to provide a new, improved flavour intense hydrolysate of a protein substrate, as well as a process for producing such hydrolysate.

The object of the present invention is achieved by the subject matter of the independent claims. The dependent claims further develop the idea of the present invention.

Accordingly, the present invention provides in a first aspect a hydrolysate obtainable by hydrolysing a substrate comprising at least one animal non-milk protein by a food-grade bacterium in an environment of less than 2 wt%, preferably less than 1 wt%, and more preferably less than 0.5%, salt content, wherein the food-grade bacterium is Bacillus natto.

In a second aspect, the invention pertains to a process for producing a hydrolysate comprising the steps of: a) mixing a substrate with a food-grade bacterium; and b) incubating the resulting mixture under conditions of less than 2 wt%, preferably less than 1 wt%, and more preferably less than 0.5 wt%, salt content; wherein the substrate comprises at least one animal non-milk protein; and wherein the food-grade bacterium is Bacillus natto.

A still further aspect of the invention is a food product comprising the hydrolysate as described herein or the hydrolysate obtainable from the process as described herein.

The inventors surprisingly found that when hydrolysing a substrate comprising proteins of an animal source such as for example fish or shrimp, with a food-grade bacterium such as for example a *Bacillus subtilis,* or particularly with *Bacillus natto,* under environmental conditions of low salt concentration, new and very intensive, pungent flavour notes can be generated which are particularly well suited for e.g. the production of seasonings or cooking aids for the preparation of Asian food dishes, while fully maintaining the microbiological safety profile of the hydrolysate during the production process. There was no need to add any anti-microbial substances such as alcohol or other preservatives into the process, or alternatively conducting the process under strict sterile conditions. Hence, new food-grade hydrolysates can be produced by this process, which have a much more intensive and pungent flavour profile as what could be achieved with the currently known processes. Thereby, for example, a very intense but typical fermented fish flavour can be produced when using sea food material as part of the substrate. Thereby, the hydrolysate may advantageously be used to produce more flavour intensive seasoning or cooking aid products, leading to more flavour rich final food products. Alternatively, the hydrolysate may be applied in lower amounts to a food product and still provide a similar flavourful food as achieved by the state of the art technologies.

The process is simple and faster than many of the alternative processes known in the art. No expensive technical installations are needed to for example keeping the process sterile. Furthermore, the production time of those hydrolysates is relatively fast, which allows to reduce the costs of such production at industrial scale. No additives such as alcohol or other chemicals are needed neither, which allows to produce an all natural hydrolysate, which is very much appreciated by the today's consumers world-wide.

The above mentioned and other features and objects of this invention will become more apparent and better understood by reference to the following detailed description. It should be understood that the detailed description made known below is not intended to be exhaustive or limit the invention to the precise form disclosed. The present invention covers all the relevant modifications and alterations made in the detailed description, unless the claims expressly state otherwise.

The present invention discloses a hydrolysate of a substrate comprising at least one animal non-milk protein, which is hydrolysed by a food-grade bacterium in an environment with salt content of less than 2 wt%, wherein the food-grade bacterium is Bacillus Natto. Preferably, the substrate is hydrolysed in an environment with salt content of less than 1 wt%, and more preferably in an environment with salt content of less than 0.5 wt%.

The term "hydrolysate" described herein refers to the product which results from hydrolysis of a substrate. The term "environment" described herein refers to the surrounding conditions of the substrate including the food-grade bacterium during the hydrolysis process.

The hydrolysate with low salt content is a notable feature of this invention, as the substrate can be hydrolysed at an optimum condition, e.g. for the food-grade bacterium to actively metabolize as well as for proteolytic enzymes released from those bacteria to be active. Furthermore, the low salt content of the hydrolysate may be suitable for many consumers, including those people that require low salt diet or prefer a healthier diet.

In an embodiment, the hydrolysis of the substrate is submerged. Thereby, the process is carried out in a submerged state, where sufficient water is added to make sure that the substrate is hydrolysed by the food-grade bacterium below the water surface. This basically assures a better mixture and a more homogenous distribution of the food-grade bacterium with the substrate, resulting in an improved hydrolysis of the substrate.

The selection of a suitable substrate is actually based on the desired characteristics attained by the hydrolysate at the end of the process, specifically in terms of organoleptic properties and nutritional values. Accordingly, the present invention pertains to a substrate comprising an animal non-milk protein, which is selected from a farm animal such as poultry or cattle, or a sea food animal such as shrimp, fish or shellfish. The invention pertains to a non-milk protein substrate. Optionally, the substrate further comprises plant proteins. The plant protein may be selected from beans such as soy beans, wheat, corn, rice, or another crop plant.

The addition of plant material to the hydrolysis process has the advantage that it may allow better conditions for a certain food-grade bacterium to grow and/or metabolise by providing for example further nutrients to those microorganisms. Thereby, the bacterial metabolic activity, and with that the overall hydrolysis process, may be faster and more efficient. Furthermore, the presence of plant material in the hydrolysis process may lead to additionally other and different flavour notes which cannot be achieved with only animal protein sources, and which add to the richness of the overall flavour profile of the final hydrolysate.

The presence of the food-grade bacterium is one of the essential features of the present invention. Generally, all types of food-grade bacterium can be employed in this invention. Preferably, the food-grade bacterium must be able to produce at least one type of protease, which is the key component needed to hydrolyse the substrate. In a preferred embodiment of the present invention, the food-grade bacterium is *Bacillus natto.*

The inventors have found that *Bacillus subtilis,* and particularly its sub-type *Bacillus natto,* are well suited for hydrolysing a substrate comprising an animal protein in a low salt environment. It is the combination of such a *Bacillus subtilis* bacterium with an animal protein substrate, specifically if from fish or shrimp, that very interesting, strong and pungent new flavours are generated. Those flavours are clearly more pronounced and stronger than the flavours generated from those substrates with the cited prior art technologies.

Furthermore, hydrolysis of said substrates with a *Bacillus subtilis* strain is fast and efficient. There is no need to provide the substrate, either prior or during the hydrolysing step with *Bacillus subtilis,* with any other hydrolysing factor such as an acid, a proteolytic enzyme, a fungus, a mold such as e.g. Aspergillus, or yeast. Therefore, a preferred embodiment of the invention is a hydrolysate which does not comprise a mold or a yeast.

The present invention also pertains to a process for producing a hydrolysate, which comprises at least the steps of a) mixing a substrate, which comprises at least one animal non-milk protein, with a food-grade bacterium, wherein the food-grade bacterium is Bacillus natto, and b) incubating the resulting mixture under conditions of less than 2 wt% salt content. The term "incubating" described herein refers to maintaining the mixture for a certain time and under certain conditions and temperature to be specified, in order to promote a particular reaction. In this specific case, the particular reaction is hydrolysis of the substrate. Preferably, the incubation is carried out under conditions of less than 1 wt% salt content, and more preferably under conditions of less than 0.5 wt% salt content. Optionally, the substrate can further comprise at least one plant protein.

In an embodiment of the present invention, the food-grade bacterium is provided to the mixture to be incubated in the form of a fermented product comprising said bacterium. The advantage is that the bacterium does not need to be specifically prepared in the form of a pure culture and possibly concentrated up for being applied into the process of the invention. In fact, the bacterium can be grown and multiplied first in a pre-culture such as in a regular fermentation of a selected substrate, and thereafter mixed into the process of the invention in the form of that fermented selected substrate. Thereby, the bacterium is usually still in a very active metabolic state and present in a high concentration, which allows an optimal and fast start of the hydrolysis process of the invention. In a preferred embodiment of the invention, the fermented product is obtainable by a solid-state fermentation of cooked soy beans, wheat gluten, or a combination thereof, with *Bacillus natto.*

In an embodiment, water is added to the mixture of the process of the invention. The addition of water to the mixture can be done either during the mixing step of the substrate with the food-grade bacterium, or during the incubation step. Thereby, a submerged hydrolysis of the substrate can be achieved.

The mixture is incubated for 8 to 48 hours in a temperature range from 45 °C to 65 °C. Preferably, the mixture is incubated for 15 to 25 hours in said temperature range. These conditions allow to optimally hydrolyse the substrate and to generate the desired flavour, taste and texture qualities of the hydrolysate.

Optionally, a lactic acid bacterium can be added to the process during the mixing or the incubation step. The addition may cause a further increase in the nutritional value of the hydrolysate. Further, this may have a positive impact on the organoleptic property of the hydrolysate as well, as some of the released amino acids will be converted into aroma compounds. In this specific embodiment, the parameters for the incubation step need to be adapted to the growth conditions of the lactic acid bacterium. Therefore, the mixture of the substrate with the lactic acid bacterium is further incubated for 10 to 25 hours in a temperature range from 25 °C to 45 °C.

The lactic acid bacterium that is suitable for addition in said process can be selected from the genus of *Lactobacillus, Leuconostoc, Pediococcus, Streptococcus, Enterococcus*, or a combination thereof. For instance, *Lactobacillus sake,* which belongs to the genus of *Lactobacillus* can be used for this purpose.

The process of the invention can further comprise a step of milling the mixture, wherein the step can be carried out either during the stage of mixing the substrate with the food-grade bacterium, or during the stage of incubation. The advantage is that a better mixing between the substrate and the bacterium can be achieved, which allows an improved and faster hydrolysis process. Furthermore, the end-product of the hydrolysis process has a much smoother appearance and mouth feel after the step of milling the mixture.

The hydrolysate and the hydrolysate obtainable from the production process according to the present invention can be used directly or further processed to obtain a food product, which may be a cooking base, seasoning, culinary aid or other edible product. Thereby, advantageously, such a food product has a new, superior and intense flavour and taste profile, whereby the achieved flavour and taste is the result of an all natural hydrolysis process.

Those skilled in the art will understand that they can freely combine all features of the present invention disclosed herein. In particular, features described for the product of the present invention may be combined with the process of the invention, and vice versa. Features described for the different embodiments of the present invention may be combined.

The present invention will now be further explained by the following examples. The examples are intended for assisting in understanding the core aspects of the present invention, including its embodiments. The present invention is not in any way limited by these examples.

### Example 1

250 kg of whole soy bean was cleaned and coarsely crushed into small pieces in a mill. The crushed soy bean was soaked in 250 kg water at 60 °C for 30 minutes in a rotating cooker. The soaked soy bean was then heated to 124 °C and held at the same temperature for 2 minutes. Afterwards, it was cooled to below 40 °C by applying vacuum. The pasteurisation step was carried out to eliminate contamination and promote protein denaturation. The cooked, cooled soy bean was discharged from the cooker and inoculated with a starter culture of *Bacillus natto.* The mixture was transported to a fermentor via a screw conveyor, where it was evenly distributed over the fermentor floor resulting in a bed of 30 cm depth. During the 72 hours of cultivation, air at 40 °C was circulated through the bed. This air was humidified to near saturation with an air conditioning unit. The water of the conditioning unit was changed every day. The ceiling of the fermentor was kept at 50 °C to prevent condensation of humid air on the surface. Any condensation in the air duct or at the bottom of the bed was drained away.

At the end of fermentation, the fermented product was discharged and 60 kg of the fermented product was added into a 250 L jacketed stirred tank containing water at 55 °C. The water was added to the fermented product in a weight ratio of 2:1. Dried baby shrimp was then added into the hydrolysis tank containing the fermented product at a weight ratio of 1:3 of dried shrimp versus fermented product.

After 1 hour of hydrolysis, the mixture was milled by a colloidal mill. After the milling, the hydrolysis was continued for another 20 hours to achieve a higher degree of hydrolysis. After the hydrolysis, salt was added at 14% wt/wt to the hydrolysate and mixed homogenously. This was to retard the growth of microorganisms upon storage. The hydrolysate was pasteurised at 140 °C for 15 seconds before filling into the plastic container for storage.

The end product was found to have excellent body and with a robust rounded seafood flavour profile. Specifically, the end product attained the traditional dawadawa odour. The end product was formulated with other fresh ingredients to form a culinary aid.

### Example 2

60kg of fresh fish was first rinsed with water to eliminate foreign matter. The fish was then loaded onto the mincer with a screen size of 3 mm. Minced fish was mixed with 60 kg of roasted wheat flour with a blender. The dough was then extruded with the meat mincer to form cylindrical rod-like substrates with a diameter of 5 mm. The substrate was autoclaved at 100°C for 20 minutes. The cooked, cooled fish was discharged from the autoclave and inoculated with a starter culture of *Bacillus natto.* This was then fermented at 40 °C for 72 hours. The surrounding air was humidified to near saturation with an air conditioning unit. The water of the conditioning unit was changed every day. The ceiling of the fermentor was kept at 50 °C to prevent condensation of humid air on the surface. Any condensation in the air duct or at the bottom of the bed was drained away.

At the end of fermentation, the fermented product was discharged from the fermentor. 80 kg of the fermented product was added into a 250L jacketed stirred tank containing water at 55 °C. The water to fermented product weight ratio was set at 3:2. After 1 hour of hydrolysis, the mixture was milled by a colloidal mill. After the milling, the hydrolysis was continued for another 20 hours to achieve a higher degree of hydrolysis. Thereafter, the hydrolysate was pasteurised at 140 °C for 15 seconds.

The end product was found to have excellent body and with a robust rounded seafood flavour profile. The end product was formulated with other fresh ingredients to form a culinary aid.

### Example 3

A similar procedure to that described in Example 1 was followed except that the soy bean was replaced with pelletised wheat gluten. The pelletised wheat gluten was obtained by extruding vital wheat gluten powder in an extruder to form pellets with a particulate size of approximately 5 mm. 250 kg of wheat gluten pellets were soaked in 250 kg water at 60 °C for 30 minutes in a rotating cooker. The soaked wheat gluten pellets were then heated to 100 °C and held at the same temperature for 10 minutes. Thereafter, it was cooled to below 40 °C by applying vacuum. The cooked, cooled wheat gluten pellets were discharged from the cooker and inoculated with a starter culture of *Bacillus natto.* The fermentation and hydrolysis followed that of Example 1.

The end product was found to have a higher glutamic acid level compared to the end product produced in Example 1. It had an excellent body and a rounded flavour profile. The end product was formulated with sugar, spices, etc to form a culinary aid.

### Example 4

A similar procedure to that described in Example 1 was followed except that the duration of hydrolysis at 55°C was shortened to 8 hours, and after which, the hydrolysis temperature was lowered to 35 °C. 1 kg of a broth culture of *Lactobacillus sake* was added when the hydrolysate temperature reached 35 °C. The hydrolysis was continued for another 12 hours at 35 °C before salt addition (12% wt/wt) and pasteurisation at 140°C for 20 seconds.

### Example 5

A similar procedure to that described in Example 1 was followed except that the dried baby shrimp was replaced with fresh minced fish. The fresh minced fish was added to the fermented product in a weight ratio of 1:1, and the water was added to the fermented product in a weight ratio of 3:2.

The end product was found to have excellent body and a rounded flavour profile. The end product was formulated with sugar, spices, etc to form a culinary aid.

## Claims

1. A hydrolysate obtainable by hydrolysing a substrate comprising at least one animal non-milk protein by a food-grade bacterium in an environment of less than 2 wt% salt content, wherein the food-grade bacterium is *Bacillus natto.*

2. The hydrolysate in accordance with claim 1, wherein hydrolysing said substrate is in an environment of less than 1 wt% salt content, preferably of less than 0.5 wt% salt content.

3. The hydrolysate in accordance with any one of claims 1 to 2, wherein hydrolysing the substrate is submerged.

4. The hydrolysate in accordance with any one of claims 1 to 3, wherein the animal protein is selected from farm animals comprising poultry, or sea food animal comprising shrimp or fish.

5. The hydrolysate in accordance with any one of claims 1 to 4, wherein the substrate further comprises at least one plant protein.

6. The hydrolysate in accordance with claim 5, wherein the plant protein is selected from beans such as soy bean, wheat, corn or rice.

7. A process for producing a hydrolysate, comprising the steps of:
a) mixing a substrate with a food-grade bacterium; and
b) incubating the mixture of step a) under conditions of less than 2 wt% salt content;
wherein the substrate comprises at least one animal non-milk protein; and wherein the food-grade bacterium is *Bacillus natto.*

8. The process in accordance with claim 7, wherein the substrate further comprises at least one plant protein.

9. The process in accordance with any one of claims 7 to 8, wherein the food-grade bacterium is provided to step a) in the form of a fermented product comprising said bacterium.

10. The process in accordance with claim 9, wherein the fermented product comprising said bacterium is obtainable by solid-state fermentation of cooked soy beans, wheat gluten, or a combination thereof, with *Bacillus natto.*

11. The process in accordance with any one of claims 7 to 10, wherein water is added to the mixture in step a).

12. The process in accordance with any one of claims 7 to 11, wherein step b) is carried out from 8 to 48 hours, preferably from 15 to 25 hours, in a temperature range from 45 °C to 65 °C.

13. A food product comprising the hydrolysate in accordance with any one of claims 1 to 6, or the hydrolysate obtainable from the process in accordance with any one of claims 7 to 12.

## Patentansprüche

1. Hydrolysat erhältlich durch Hydrolysieren eines Substrats aufweisend mindestens ein nicht aus Milch stammendes Tierprotein durch ein Bakterium in Lebensmittelqualität in einer Umgebung von weniger als 2 Gew.-% Salzgehalt, wobei das Bakterium in Lebensmittelqualität *Bacillus natto* ist.

2. Hydrolysat nach Anspruch 1, wobei das Hydrolysieren des Substrats in einer Umgebung von weniger als 1 Gew.-% Salzgehalt, vorzugsweise von weniger als 0,5 Gew.-% Salzgehalt, stattfindet.

3. Hydrolysat nach einem der Ansprüche 1 bis 2, wobei das Hydrolysieren des Substrats in einem eingetauchten Zustand erfolgt.

4. Hydrolysat nach einem der Ansprüche 1 bis 3, wobei das Tierprotein ausgewählt ist aus Nutztieren, aufweisend Geflügel, oder Meerestiere, aufweisend Garnelen oder Fisch.

5. Hydrolysat nach einem der Ansprüche 1 bis 4, wobei das Substrat weiterhin mindestens ein Pflanzenprotein aufweist.

6. Hydrolysat nach Anspruch 5, wobei das Pflanzenprotein ausgewählt ist aus Bohnen wie Sojabohnen, Weizen, Mais oder Reis.

7. Verfahren zur Herstellung eines Hydrolysats, aufweisend die Schritte:
a) Mischen eines Substrats mit einem Bakterium in Lebensmittelqualität; und
b) Inkubieren der Mischung von Schritt a) unter Bedingungen mit weniger als 2 Gew.-% Salzgehalt;
wobei das Substrat mindestens ein nicht aus Milch stammendes Tierprotein aufweist; und
wobei das Bakterium in Lebensmittelqualität *Bacillus natto* ist.

8. Verfahren nach Anspruch 7, wobei das Substrat weiterhin mindestens ein Pflanzenprotein aufweist.

9. Verfahren nach einem der Ansprüche 7 bis 8, wobei das Bakterium in Lebensmittelqualität für Schritt a) in Form eines fermentierten Produktes aufweisend das Bakterium bereitgestellt ist.

10. Verfahren nach Anspruch 9, wobei das fermentierte Produkt aufweisend das Bakterium durch Festphasenfermentation von gekochten Sojabohnen, Weizenkleber oder eine Kombination daraus mit *Bacillus natto* erhältlich ist.

11. Verfahren nach einem der Ansprüche 7 bis 10, wobei Wasser der Mischung in Schritt a) zugefügt wird.

12. Verfahren nach einem der Ansprüche 7 bis 11, wobei Schritt b) von 8 bis 48 Stunden, vorzugsweise von 15 bis 25 Stunden, in einem Temperaturbereich von 45°C bis 65°C durchgeführt wird.

13. Lebensmittelprodukt aufweisend das Hydrolysat nach einem der Ansprüche 1 bis 6, oder das Hydrolysat erhältlich aus dem Verfahren nach einem der Ansprüche 7 bis 12.

## Revendications

1. Hydrolysat susceptible d'être obtenu par hydrolyse d'un substrat comprenant au moins une protéine non laitière animale par une bactérie de qualité alimentaire dans un environnement de moins de 2% en poids de teneur en sel, dans lequel la bactérie de qualité alimentaire est *Bacillus natto.*

2. Hydrolysat selon la revendication 1, dans lequel l'hydrolyse dudit substrat a lieu dans un environnement de moins de 1% en poids de teneur en sel, de préférence de moins de 0,5% en poids de teneur en sel.

3. Hydrolysat selon l'une quelconque des revendications 1 à 2, dans lequel l'hydrolyse du substrat est immergée.

4. Hydrolysat selon l'une quelconque des revendications 1 à 3, dans lequel la protéine animale est choisie parmi les animaux d'élevage comprenant la volaille, ou les animaux marins comprenant crevette ou poisson.

5. Hydrolysat selon l'une quelconque des revendications 1 à 4, dans lequel le substrat comprend en outre au moins une protéine végétale.

6. Hydrolysat selon la revendication 5, dans lequel la protéine végétale est choisie à partir de haricots, fèves et féveroles comme par exemple le soja, le blé, le maïs ou le riz.

7. Procédé de production d'un hydrolysat, comprenant les étapes consistant à :
a) mélanger un substrat avec une bactérie de qualité alimentaire ; et
b) incuber le mélange de l'étape a) dans des conditions de moins de 2% en poids de teneur en sel ;
dans lequel le substrat comprend au moins une protéine non laitière animale ; et dans lequel la bactérie de qualité alimentaire est *Bacillus natto.*

8. Procédé selon la revendication 7, dans lequel le substrat comprend en outre au moins une protéine végétale.

9. Procédé selon la revendication 7 ou 8, dans lequel la bactérie de qualité alimentaire est fournie à l'étape a) sous la forme d'un produit fermenté comprenant ladite bactérie.

10. Procédé selon la revendication 9, dans lequel le produit fermenté comprenant ladite bactérie est susceptible d'être obtenu par fermentation en milieu solide de soja cuit, gluten de blé, ou combinaison de ceux-ci, avec *Bacillus natto.*

11. Procédé selon l'une quelconque des revendications 7 à 10, dans lequel de l'eau est ajoutée au mélange dans l'étape a).

12. Procédé selon l'une quelconque des revendications 7 à 11, dans lequel l'étape b) est réalisée de 8 à 48 heures, de préférence de 15 à 25 heures, dans une plage de température de 45 °C à 65 °C.

13. Produit alimentaire comprenant l'hydrolysat selon l'une quelconque des revendications 1 à 6, ou l'hydrolysat susceptible d'être obtenu à partir du procédé selon l'une quelconque des revendications 7 à 12.
